# FASCICULE DE BREVET EUROPEEN

(11) **EP 2 794 170 B1**
(45) Date de publication et mention de la délivrance du brevet: **25.03.2020**
(21) Numéro de dépôt: 12806505.9
(22) Date de dépôt: 21.12.2012
(51) Int. Cl.: B23K 1/19, A61L 27/08, B23K 35/30, B23K 35/32

(54) **PROCÉDÉ D'ASSEMBLAGE PAR BRASAGE D'UN SUBSTRAT COMPRENANT DU PYROCARBONE AVEC DES PIÈCES COMPRENANT DU PYROCARBONE.**
VERFAHREN ZUR MONTAGE EINES PYROKOHLENSTOFFHALTIGEN SUBSTRATS MIT PYROKOHLENSTOFFHALTIGEN TEILEN DURCH LÖTEN
METHOD FOR ASSEMBLING, BY BRAZING, A SUBSTRATE COMPRISING PYROCARBON WITH PARTS COMPRISING PYROCARBON

(30) Priorité: 22.12.2011 FR 1162338
(43) Date de publication de la demande: 29.10.2014
(73) Titulaire: Commissariat à l'Énergie Atomique et aux Énergies Alternatives, 75015 Paris (FR)
(72) Inventeur: CHAUMAT, Valérie, F-38760 Saint-paul De Varces (FR); BUCCI, Philippe, F-38360 Engins (FR); MAILLIART, Olivier, F-14000 Caen (FR)
(74) Mandataire: Brevalex
(86) Numéro de dépôt international: PCT/EP2012/076751
(87) Numéro de publication internationale: WO 2013/093052

(56) Documents cités:
- WO-A1-2010/137651
- DE-A1- 2 759 148
- FR-A1- 2 729 322
- FR-A1- 2 893 247
- US-A- 3 609 856

## Description

### DOMAINE TECHNIQUE

La présente invention est relative à un procédé d'assemblage par brasage, d'un substrat comprenant du pyrocarbone avec des pièces comprenant du pyrocarbone. Un procédé d'assemblage par brasage de pièces en carbone est par exemple connu du document US 3609856 A1.

Plus précisément, l'invention concerne un procédé d'assemblage par brasage d'un substrat comprenant au moins une surface externe en pyrocarbone avec des pièces, aussi appelées éléments rapportés, comprenant chacune au moins une surface externe en pyrocarbone.

Le substrat et les éléments rapportés, tels que des billes, peuvent être constitués de pyrocarbone, ou bien ils peuvent être constitués par un premier matériau ou matériau de cœur, généralement biocompatible, recouvert d'une peau en pyrocarbone.

Le procédé d'assemblage selon l'invention est généralement mis en œuvre à une température de brasage ne dépassant pas 1700°C, de préférence entre environ 1000°C et 1450°C.

Le procédé selon l'invention permet notamment de réaliser des implants destinés à être introduits dans un patient.

Le domaine technique de l'invention peut donc être défini comme celui des implants comprenant du pyrocarbone.

### ÉTAT DE LA TECHNIQUE ANTÉRIEURE

Le pyrocarbone est biocompatible et est utilisé comme implant, ou partie d'implant, pour les prothèses de parties osseuses. Il est également utilisé comme implant d'interposition mobile entre deux surfaces osseuses et assure la fonction d'articulation.

Il est particulièrement intéressant pour cette fonction car il présente un très bon coefficient de frottement avec l'os et un module de Young voisin de celui de l'os, ce qui évite d'engendrer une usure de l'os et de l'implant.

Dans la littérature, les implants de type prothèse, pour remplacer des os ou parties d'os sont en général composés de deux parties, une pièce mâle et une pièce femelle. L'une au moins de ces pièces comporte une tige qui vient s'engager dans une partie osseuse adjacente à l'os ou partie d'os que l'on souhaite remplacer, afin de maintenir la prothèse contre la partie osseuse. Ces opérations de fixation de la prothèse sont délicates à mettre en œuvre et obligent à faire un trou dans l'os pour le passage de la tige. Ces fixations ne laissent pas la possibilité au chirurgien de remplacer la prothèse en cas d'usure, sauf à créer des dommages conséquents sur le ou les os auxquels la tige est fixée.

Ainsi le document [1] décrit un implant comprenant une tête en pyrocarbone, destinée à remplacer au moins une partie articulaire latérale d'un os recevant des charges transversales et un élément de maintien connecté à la tête et destiné à être fixé audit os.

Il existe donc un besoin pour des implants qui pourraient être fixés sans qu'il soit nécessaire de percer l'os. La tête est mobile en translation par rapport à l'élément de maintien dans au moins une direction correspondant, lorsque l'implant est en place dans le patient, à une direction desdites charges transversales.

Des implants d'interposition entre deux surfaces osseuses sont décrits notamment dans le document [2].

Ces implants à base de pyrocarbone, en particulier dont au moins la surface externe est en pyrocarbone sont mobiles entre les surfaces osseuses.

Cela nécessite de créer un logement pour ces implants, et parfois, une ou plusieurs surfaces osseuses, plus ou moins usées, en regard de l'implant mobile, doivent être rognées de façon appropriée pour aménager ce logement.

Il existe donc un besoin pour des implants d'interposition qui ne nécessitent pas la création d'un logement, en rognant des surfaces osseuses pour les accueillir.

Le but de la présente invention est de fournir un procédé de préparation de substrat en pyrocarbone ou dont au moins la surface externe est en pyrocarbone, notamment destiné à être utilisé comme implant qui réponde entre autres aux besoins mentionnés plus haut.

### EXPOSÉ DE L'INVENTION

Ce but, et d'autres encore, sont atteints, conformément à l'invention, par un procédé d'assemblage par brasage d'un substrat comprenant au moins une surface externe en pyrocarbone avec des pièces comprenant chacune au moins une surface externe en pyrocarbone, dans lequel on réalise les étapes successives suivantes :
- on dépose une composition de brasure constituée par un alliage d'un ou plusieurs métal (métaux) ou métalloïde(s) sur au moins une partie à assembler de la surface externe en pyrocarbone du substrat ;
- on dépose les pièces sur la composition de brasure, de façon à ce que la composition de brasure, lors de sa fusion, soit en contact d'une part avec la partie à assembler de la surface externe du substrat et d'autre part avec une partie à assembler de la surface externe en pyrocarbone de chacune des pièces, de façon à ce que la forme des pièces ne soit pas modifiée et reste apparente, de façon à laisser des espaces libres, vides entre les pièces, et de façon à ce que les pièces ne soient pas noyées dans la composition de brasure ;
- on chauffe l'ensemble formé par le substrat, la composition de brasure et les pièces déposées, sous vide ou sous une atmosphère de gaz neutre, à une température de brasage suffisante pour faire fondre totalement ou au moins partiellement la composition de brasure, et inférieure à 1700°C ;
- on refroidit le substrat, la composition de brasure et les pièces jusqu'à la solidification de la composition de brasure,
procédé dans lequel ladite composition de brasure est choisie parmi les alliages à base de silicium contenant du Si à une concentration supérieure à 40% atomiques et qui présentent une réactivité limitée et maîtrisée avec le pyrocarbone.

La fusion de la composition de brasure est généralement considérée comme totale lorsqu'elle se trouve dans un état liquide, à une température supérieure ou égale au liquidus.

La fusion de la composition de brasure est généralement considérée comme partielle lorsqu'elle se trouve dans un état que l'on peut qualifier de semi-solide, visqueux, ramolli, à une température située entre le solidus et le liquidus.

Les pièces ne doivent pas être noyées dans une quantité importante de composition de brasure, elles sont seulement assemblées par une partie à assembler, qui est généralement leur base au substrat.

La forme globale des pièces doit rester apparente, visible, identifiable afin d'assurer un rôle de structuration de surface avec des espaces libres, vides, entre les pièces pour permettre la croissance de cellules, telles que des cellules osseuses.

Les parties des pièces qui ne sont pas dans la zone d'assemblage, autrement dit les parties de la surface externe en pyrocarbone de chacune des pièces différentes des parties à assembler de la surface externe de chacune des pièces peuvent être ou pas recouvertes de composition de brasure, à condition que l'épaisseur du recouvrement reste très faible par rapport aux dimensions de la pièce, afin de ne pas modifier la forme des pièces et de laisser libre, vide des espaces entre les pièces.

La partie à assembler de la surface externe du substrat et la partie à assembler de la surface externe de chacune des pièces sont généralement en pyrocarbone.

De préférence, la totalité de la surface externe du substrat et la totalité de la surface externe des pièces sont en pyrocarbone.

Deux ou plus parmi les pièces déposées peuvent être en contact entre elles ou bien toutes les pièces déposées peuvent ne pas être en contact entre elles.

Le procédé selon l'invention est un procédé de brasage.

On sait que le brasage est une technique qui permet d'assembler au moins deux pièces par fusion d'un matériau d'apport, appelé brasure ou alliage de brasage, dont la température de fusion est inférieure à celle des matériaux à assembler.

Le matériau à assembler selon l'invention est le pyrocarbone qui ne présente pas de point de fusion mais qui peut néanmoins être dégradé par une température supérieure à 1700°C.

Les substrats, pièces, dépôts ou revêtements en pyrocarbone sont généralement fabriqués à très haute température, par exemple vers 1400°C et ne doivent donc pas être exposés à des températures supérieures à 1700°C lors du cycle de brasage. C'est la raison pour laquelle la température de brasage mise en œuvre dans le procédé selon l'invention n'excède pas 1700°C car au-delà de cette température, le pyrocarbone serait dégradé.

Avantageusement, le brasage est effectué à une température de brasage supérieure d'au moins 15°C, de préférence d'au moins 30°C à la température de fusion de la brasure.

De préférence, la température de brasage est de 980°C à 1450°C.

Le procédé selon l'invention, qui est un procédé d'assemblage par brasage d'un substrat et de pièces spécifiques dont au moins la surface externe est en un matériau spécifique, à savoir le pyrocarbone, n'a jamais été décrit ni suggéré dans l'art antérieur.

Ainsi, le document [4] propose un procédé d'assemblage d'au moins deux pièces en matériaux à base de carbure de silicium par brasage réfractaire non réactif, dans lequel la composition de brasure est constituée de 40% à 97% atomiques de silicium et de 60% à 3% atomiques d'un autre élément parmi Cr, Re, V, Ru, Ir, Rh, Pd, Co, Pt, Ce, Zr et dans lequel, préalablement au brasage, on ajoute un renfort de SiC et/ou de C à la composition.

Ce document concerne donc spécifiquement le brasage de pièces à base de carbure de silicium.

Des compositions de brasure contenant du silicium comme celles décrites dans le document [4] peuvent être mises œuvre dans le procédé selon l'invention, mais le fait qu'une composition de brasure convienne au brasage de pièces à base de carbure de silicium ne signifie en rien que cette même composition de brasure puisse convenir au brasage de pièces en pyrocarbone.

En effet, le comportement des compositions de brasure est extrêmement difficile à prévoir et est hautement aléatoire notamment en fonction du matériau constituant les pièces à assembler.

Le fait qu'une composition de brasure soit apte au brasage de pièces en un matériau donné et possède d'excellentes propriétés et donne d'excellents résultats pour le brasage de pièces en ce matériau donné ne signifie en rien que cette même composition possédera les mêmes propriétés et donnera les mêmes résultats ou sera même apte au brasage de pièces en un autre matériau.

En particulier, il était absolument impossible au regard du document [4] de prévoir le bon comportement des interfaces entre le pyrocarbone et les alliages de brasage de ce document, et en particulier le comportement mécanique au niveau de ces interfaces.

Le document [5] décrit un procédé de recouvrement de pièces en matériaux à base de carbure de silicium à l'aide d'une composition de recouvrement constituée par une composition de brasure choisie parmi les mêmes compositions que celles mentionnées dans le document [4], à laquelle est ajouté un renfort de SiC et/ou de C.

Là-encore, les pièces recouvertes sont exclusivement des pièces à base de SiC et non des pièces en pyrocarbone.

De plus, les dépôts préparés dans le document [5] sont couvrants et très homogènes dans le but de protéger la céramique à base de SiC.

Selon l'invention, on ne forme pas un revêtement homogène et protecteur mais on réalise plutôt une structuration de la surface externe en pyrocarbone d'un implant en formant des aspérités et trous entre les éléments rapportés brasés, par exemple les billes brasées, par exemple au niveau de leur base.

Le procédé selon l'invention permet notamment de préparer des implants avec un substrat en pyrocarbone ou revêtu de pyrocarbone pourvu d'une surface structurée sur laquelle les cellules osseuses vont pouvoir se développer.

La surface structurée est selon l'invention constituée par des pièces, éléments rapportés, tels que des billes, recouverts de pyrocarbone et assemblés par brasage sur la surface externe du substrat. Cette surface structurée se trouve en vis-à-vis de l'os lorsque l'implant est positionné dans le patient.

Le procédé selon l'invention permet de préparer des implants qui ne présentent pas les inconvénients des implants de l'art antérieur et qui permettent de résoudre les problèmes des implants de l'art antérieur.

Ainsi en ce qui concerne les implants de type prothèse, le procédé selon l'invention permet de réaliser une surface structurée sur le substrat en pyrocarbone ou à surface en pyrocarbone.

Cette structuration est obtenue par l'assemblage par brasage des éléments rapportés, par exemples des billes, en pyrocarbone ou revêtus de pyrocarbone sur le substrat.

Ainsi, il n'est pas utile de percer l'os pour fixer l'implant. La partie osseuse défectueuse est usinée et/ou légèrement polie, l'implant est placé sur l'os avec la partie structurée orientée vers l'os. L'ensemble est maintenu sans bouger pendant la phase au cours de laquelle les cellules osseuses se développent dans la partie structurée jusqu'à rendre solidaire l'os et l'implant. Cela évite ainsi de percer l'os.

Pour ce qui est des implants d'interposition, le procédé selon l'invention permet de préparer des implants structurés formés d'une surface externe en pyrocarbone sur laquelle sont assemblés par brasage des éléments rapportés, par exemple des billes, en pyrocarbone ou recouverts de pyrocarbone.

Les surfaces osseuses en regard de l'implant sont simplement légèrement usinées et polies et il n'est plus nécessaire de rogner de manière importante ces surfaces osseuses pour y aménager un logement.

Les implants préparés par le procédé selon l'invention avec leur surface structurée en pyrocarbone sont ensuite placés sur ces surfaces légèrement usinées et polies.

La composition de brasure, alliage de brasage mise en œuvre selon l'invention doit permettre de réaliser un palier de brasage à une température maximale de 1700°C sous vide secondaire ou sous atmosphère neutre.

Ensuite, l'alliage de brasage doit aussi présenter une réactivité limitée et maîtrisée tout en formant des liaisons fortes avec le pyrocarbone.

Les inventeurs ont mis en évidence que, de manière surprenante, des alliages de brasage connus pour le brasage d'autres matériaux que le pyrocarbone convenaient pour le brasage du pyrocarbone.

Ainsi, la composition de brasure va être choisie parmi les alliages à base de silicium contenant du Si à une concentration supérieure à 40% atomiques et qui présentent une réactivité limitée et maîtrisée avec le pyrocarbone (troisième catégorie d'alliages de brasage).

Précisons que par « alliage présentant une réactivité limitée avec le pyrocarbone », on entend généralement qu'il y a dissolution du carbone et formation de SiC dans une zone dont l'épaisseur est inférieure à 20 µm, voire à 10 µm, donc d'une épaisseur généralement très faible vis-à-vis de l'épaisseur de la couche de pyrocarbone.

Précisons que par « alliage présentant une réactivité maîtrisée avec le pyrocarbone », on entend généralement que la réaction entre l'alliage et le pyrocarbone est reproductible et sensiblement identique d'une pièce, telle qu'une bille, à l'autre.

Les compositions de brasure de la troisième catégorie peuvent être choisies parmi les alliages binaires constitués par du silicium à une concentration supérieure à 40% atomiques et par un élément choisi parmi Cr, Re, V, Ru, Ir, Rh, Pd, Co, Pt, Ce, Zr, Ti, Ag, Au, Cu, Nd, Pr, Y, et Hf, c'est-à-dire les alliages binaires SiCr, SiRe, SiV, SiRu, Silr, SiRh, SiPd, SiCo, SiPt, SiCe, SiZr, SiTi, SiAg, SiAu, SiCu, SiNd, SiPr, SiY, et SiHf.

De préférence, les compositions de brasure appartenant à la troisième catégorie peuvent être choisies parmi les alliages binaires (A) constitués de silicium et de zirconium, les alliages binaires (B) constitués de silicium et de titane, les alliages binaires (C) constitués de silicium et de cobalt, et les alliages binaires (D) constitués de silicium et d'argent.

Avantageusement, l'alliage binaire (A) est constitué en pourcentages atomiques de 75% à 97% de silicium, et de 25% à 3% de Zr ; de préférence de 89% à 97% de silicium, et de 11% à 3% de zirconium.

Avantageusement, l'alliage binaire (B) est constitué en pourcentages atomiques de 60% à 97% de silicium, et de 40% à 3% de Ti ; de préférence de 80% à 97% de silicium, et de 20% à 3% de titane.

Avantageusement, l'alliage binaire (C) est constitué en pourcentages atomiques de 60% à 97% de silicium, et de 40% à 3% de Co ; de préférence de 75% à 97% de silicium, et de 25% à 3% de cobalt.

Avantageusement, l'alliage binaire (D) est constitué en pourcentages atomiques de plus de 40 % à 97% de silicium, et de moins de 60% à 3% d'Ag ; de préférence de plus de 40 % à 90% de silicium, et de moins de 60% à 10% d'argent.

Avantageusement, le substrat est constitué par du pyrocarbone, ou bien le substrat est constitué par un cœur en un premier matériau choisi de préférence parmi les matériaux biocompatibles tels que la zircone, le graphite, ou le carbure de silicium ; au moins une partie de la surface externe du cœur en un premier matériau, et de préférence la totalité de la surface externe du cœur en un premier matériau étant revêtue par un revêtement ou peau en pyrocarbone.

Avantageusement, les pièces sont constituées par du pyrocarbone ou bien chacune des pièces est constituée par un cœur en un premier matériau choisi de préférence parmi les matériaux biocompatibles tels que la zircone, le graphite, ou le carbure de silicium ; au moins une partie de la surface externe du cœur en un premier matériau et de préférence la totalité de la surface externe du cœur en un premier matériau étant revêtue par un revêtement ou peau en pyrocarbone.

Avantageusement, les pièces se présentent sous la forme de billes sphériques ou sphéroïdales, de préférence avec une granulométrie comprise entre 2,5 mm et quelques dizaines de micromètres, par exemple entre 2,5 mm et 100 µm, de préférence entre 2,5 mm et 50 µm.

Avantageusement, on peut former une poudre de composition de brasure, on met en suspension ladite poudre dans un liant organique de façon à obtenir une suspension ou pâte de composition de brasure, et on dépose ladite suspension ou pâte sur au moins une partie à assembler de la surface externe en pyrocarbone du substrat.

Avantageusement, ledit substrat est un implant, ou partie d'implant, pour prothèses de parties osseuses ; ou un implant d'interposition entre des surfaces osseuses.

Le procédé selon l'invention, notamment dans le cas où les alliages de brasage préférés A, B, C, et D sont mis en œuvre, assure que les pièces ou éléments rapportés, tels que des billes, et le substrat, conservent toute leur intégrité à l'issue de l'opération d'assemblage.

Le procédé selon l'invention, notamment dans le cas où les alliages de brasage préférés A, B, C, et D sont mis en œuvre, permet d'obtenir, entre autres, les propriétés suivantes :
- un bon mouillage de la surface des pièces, éléments rapportés, tels que des billes, qui se trouve en contact avec le substrat et du substrat, par les alliages de brasage.

Cette surface des pièces qui se trouve en contact avec le substrat est généralement définie comme la base de ces pièces telles que des billes.

Cela conduit ainsi à une bonne surface d'accroche des pièces, ou éléments rapportés tels que des billes avec par exemple une remontée de la brasure sur les bords de la bille et un bon remplissage du joint entre la bille et le substrat, ce qui améliore le comportement mécanique en permettant un meilleur transfert des contraintes.

Toutefois, selon l'invention, les éléments rapportés, pièces, tels que des billes ne sont pas « noyés » dans la brasure (c'est-à-dire la composition de brasure solidifiée après fusion) mais seulement accrochés par une de leur surface ou base, au substrat afin de créer une surface structurée c'est-à-dire avec des zones en relief, en saillie, des aspérités, des zones en creux, et des espaces libres, vides entre les pièces; et/ou à forte rugosité ; qui permettent la croissance de cellules telles que des cellules osseuses entre les pièces ou éléments rapportés tels que des billes.

La forme globale des éléments rapportés, pièces tels que des billes, n'est pas modifiée, demeure inchangée, apparente afin de garantir une structuration de surface avec des espaces disponibles pour la croissance des cellules telles que des cellules osseuses.

Seule la « base » des pièces est modifiée du fait de la formation d'un joint entre la pièce et le substrat.

En effet selon l'invention, on dépose les pièces sur la composition de brasure, de façon à ce que la composition de brasure lors de sa fusion soit en contact d'une part avec la surface externe du substrat et d'autre part avec une partie à assembler de la surface externe en pyrocarbone de chacune des pièces.

Les pièces, éléments rapportés peuvent être en contact entre eux, ou bien ne pas être en contact entre eux.

Lorsque les éléments, pièces rapportés ne sont pas en contact entre eux, ils peuvent être qualifiés d'éléments, pièces, distincts.

Lorsque les pièces, éléments rapportés sont en contact entre eux, il faut de toutes façons qu'il y ait de l'espace entre les éléments rapportés mais cela n'empêche pas qu'il y ait quelques points de contact entre les éléments rapportés.

Le mode préféré est celui où les pièces, éléments rapportés ne sont pas en contact entre eux.

Dans tous les cas, il existe des espaces, volumes libres, une « porosité », entre les pièces, éléments rapportés, dans lesquels la croissance des cellules, telles que des cellules osseuses peut avoir lieu.

En outre, les pièces ne sont pas totalement noyées dans une quantité importante de composition de brasure, elles sont généralement assemblées seulement par la base au substrat en pyrocarbone.

La forme globale des pièces doit rester apparente afin d'assurer le rôle de structuration et permettre ainsi la croissance des cellules telles que des cellules osseuses.

Les parties des pièces qui ne sont pas dans la zone d'assemblage peuvent être ou pas recouvertes de composition de brasure, à condition que l'épaisseur du recouvrement reste très faible par rapport aux dimensions de la pièce afin de ne pas modifier la forme des pièces. Ces pièces forment ainsi une surface présentant des saillies, reliefs, des aspérités, des creux, une rugosité qui est également favorable à la croissance des cellules, telles que des cellules osseuses.

Le procédé de brasage conduit à :
- une bonne adhérence, un bon accrochage mécanique, entre la base des pièces, éléments rapportés tels que la base des billes, et le substrat après brasage. Des liaisons fortes sont formées entre le pyrocarbone du substrat et des pièces et la brasure (c'est-à-dire la composition de brasure refroidie et solidifiée).
- une bonne tenue mécanique des joints entre les pièces, éléments rapportés tels que des billes, la brasure et le substrat.
- une réactivité très modérée de l'alliage de brasage sur les substrats en pyrocarbone ou à peau en pyrocarbone (voir exemples). Il n'y a pas de zones fragilisantes complexes et poreuses au niveau de l'interface. Seule une légère dissolution du carbone du pyrocarbone dans la brasure a été observée.

De manière générale, le procédé selon l'invention s'applique principalement à la fabrication d'implants, mais d'autres applications du procédé selon l'invention se situent par exemple dans le domaine des technologies à haute température.

L'invention concerne, en outre, l'assemblage obtenu par le procédé selon l'invention.

### BRÈVE DESCRIPTION DES DESSINS

- La Figure 1 est un graphique qui représente le cycle thermique de brasage utilisé dans les exemples 1 et 2.
   En abscisse est portée la durée t en minutes depuis le début du traitement thermique, et en ordonnée est portée la température T en °C .
- La Figure 2 est une photographie qui montre le disque de zircone revêtu de pyrocarbone n°2 de l'exemple 2, avec les billes de zircone revêtues de pyrocarbone mouillées par la composition de brasure en fusion.
- La Figure 3A est une photographie qui montre des billes assemblées sur le disque n°2 de l'exemple 2.
- La Figure 3B est une photographie qui montre des billes assemblées sur le disque n°2 de l'exemple 2.
- La Figure 3C est une photographie qui montre des billes assemblées sur le disque n°7 de l'exemple 2.
- La Figure 4 est une photographie, prise au microscope électronique à balayage (MEB) de la jonction bille/substrat-disque obtenue dans l'exemple 2.
   L'échelle indiquée sur la Figure 4 représente 300 µm.
- La Figure 5 est une photographie qui montre les billes assemblées sur le disque avec une brasure SiTi dans l'exemple 4.
- La Figure 6 est une photographie prise au microscope électronique à balayage (MEB) qui montre le joint entre une bille et le substrat obtenu dans l'exemple 4.
   L'ensemble bille, brasure et substrat est enrobé, découpé et poli avant observation au MEB. L'échelle indiquée sur la Figure 6 représente 500 µm.
- La Figure 7 est une photographie prise au microscope électronique à balayage (MEB), qui est une vue agrandie d'une partie de la photographie de la Figure 6, et qui met en évidence une légère réactivité bille/brasure et substrat/ brasure.
   L'échelle indiquée sur la Figure 7 représente 100 µm.
- La Figure 8 est une photographie prise au microscope électronique à balayage (MEB) qui montre l'assemblage entre deux billes et le substrat obtenu dans l'exemple 7.
   L'échelle indiquée sur la Figure 8 représente 1 mm.
- La Figure 9 est une photographie prise au microscope électronique à balayage (MEB), qui est une vue agrandie d'une partie de la photographie de la Figure 8 et qui montre le joint entre une bille et le substrat.
   L'échelle indiquée sur la Figure 9 représente 100 µm.
- La Figure 10 est une photographie prise au microscope électronique à balayage (MEB), qui est une vue agrandie d'une autre partie de la photographie de la Figure 8 et qui montre le joint entre une bille et le substrat.
- La Figure 11 est une photographie prise au microscope électronique à balayage (MEB) qui montre l'assemblage entre une bille et le substrat obtenu dans l'exemple 8.
   L'ensemble bille, brasure et substrat est découpé et poli, mais non enrobé, avant observation au MEB.
   L'échelle indiquée sur la Figure 11 représente 1 mm.
- La Figure 12 est une photographie prise au microscope électronique à balayage (MEB), qui est une vue agrandie d'une partie de la photographie de la Figure 11, qui montre l'interface entre le pyrocarbone et la brasure et met en évidence une légère réactivité (en gris).
   L'échelle indiquée sur la Figure 12 représente 5 µm.

### EXPOSÉ DÉTAILLÉ DE MODES DE RÉALISATION PARTICULIERS

La première étape du procédé selon l'invention consiste, tout d'abord, à préparer, élaborer une composition de brasure, en d'autres termes un alliage de brasage.

La composition de brasure est, généralement, une composition pulvérulente sous la forme d'une poudre mais elle peut aussi se présenter sous la forme de feuillards.

On décrit ci-après, deux manières de préparer la composition de brasure, mais il existe de nombreuses autres manières de préparer la composition de brasure, et les deux manières de préparer la composition de brasure qui sont plus particulièrement décrites ci-dessous le sont sans que cela ne constitue une quelconque limitation.

Ainsi selon une première manière de préparer la composition de brasure, on commence par synthétiser, tout d'abord, à partir des éléments purs, un alliage contenant les éléments de la composition de brasure dans les proportions retenues pour ces éléments dans la composition.

Les éléments purs sont les éléments Si et Zr pour la composition, alliage A préféré selon l'invention ; les éléments Si et Ti pour la composition, alliage B préféré selon l'invention ; les éléments Si et Co pour la composition, alliage C préféré selon l'invention ; les éléments Si et Ag pour la composition, alliage D préféré selon l'invention

La synthèse d'un tel alliage se fait par exemple en introduisant les éléments purs par exemple sous la forme de morceaux ou autres dans les proportions voulues pour la composition de brasure à préparer dans un creuset réfractaire, par exemple dans un creuset en alumine.

L'ensemble formé par le creuset, et les éléments de la composition de brasure placés dans le creuset, est disposé dans un appareil de chauffage, tel qu'un four en graphite ou un four métallique, et est chauffé à une température comprise entre une Température minimale Tmin et une Température maximale Tmax, pendant une durée de 10 à 60 minutes, sous vide, typiquement de 10⁻⁴ à 10⁻⁵ mbar (par exemple pour la synthèse de SiCo) ou sous une atmosphère de gaz neutre, par exemple une atmosphère d'argon (par exemple pour la synthèse de AgSi).

Une atmosphère de gaz neutre est en effet indispensable dans certaines conditions, pour certaines brasures pour éviter la vaporisation d'un composant de la brasure, par exemple dans le cas d'une composition de brasure AgSi.

Le Tableau 1 ci-dessous précise les températures Tmin et Tmax pour les différents alliages de brasage, compositions de brasure. Tmin et Tmax définissent donc le domaine, plage de températures pour l'élaboration des alliages de brasage.

**Tableau 1: Domaine de température pour l'élaboration des alliages de brasage.**

| **Alliage** | **Domaine de composition (% at. Si)** | **Tmin (°C)** | **Tmax (°C)** |
|---|---|---|---|
| **A** | 75 à 80 | 1600°C | 1650°C |
| | 80 à 85 | 1500°C | 1600°C |
| | 85 à 88 | 1450°C | 1500°C |
| | 88 à 97 | 1420°C | 1450°C |
| **B** | 60 à 75 | 1450°C | 1570°C |
| | 75 à 97 | 1380°C | 1450°C |
| **C** | 60 à 90 | 1350°C | 1400°C |
| | 90 à 97 | 1400°C | 1450°C |
| **D** | 5 à 20 | 1010°C | 1050°C |
| | 20 à 30 | 1050°C | 1200°C |
| | 30 à 50 | 1200°C | 1300°C |
| | 50 à 97 | 1300°C | 1420°C |

Ce traitement thermique de chauffage conduit à la fusion des éléments de la composition de brasure.

On obtient après refroidissement l'alliage de brasage final désiré, homogène, sous la forme d'un lingot.

Le lingot de l'alliage obtenu peut être ensuite broyé dans tout appareil adéquat, par exemple dans un mortier, pour obtenir une poudre de granulométrie adéquate, c'est-à-dire dont les grains ont, par exemple, une taille définie par leur plus grande dimension, telle que le diamètre dans le cas de grains de forme sphérique ou sphéroïdale entre 1 et 300 µm.

Cette poudre constitue la composition de brasure.

Ou bien, notamment dans le cas des alliages les plus concentrés en Ag, le lingot peut être transformé en feuillard par laminage par exemple.

Ce feuillard constitue alors la composition de brasure.

Selon une seconde manière de préparer la composition de brasure, alliage de brasage binaire mis en œuvre selon l'invention, on pèse une poudre d'un élément de la composition de brasure et une poudre d'un composé intermétallique des éléments qui constituent la composition de brasure, dans les proportions retenues pour la composition de brasure et on mélange ensuite ces poudres dans un « Turbula » pendant au moins 30 minutes.

Ainsi, dans le cas des alliages de brasage préférés A, B, et C mis en œuvre selon l'invention, on pèse en respectant les proportions retenues pour la composition de brasure de la poudre de Si, et respectivement des poudres du composé ZrSi₂ pour la composition A, TiSi₂ pour la composition B, et CoSi₂ pour la composition C, puis on mélange ces poudres.

Notons que pour la composition D, il n'existe pas de composé défini entre Ag et Si, et que dans ce cas, on mélange donc simplement des poudres de Si et d'Ag. Il y a lieu de souligner que la manipulation de la poudre d'argent doit se faire sous une atmosphère protectrice, telle qu'une atmosphère d'argon, afin d'éviter l'oxydation de cette poudre.

La poudre composée du mélange des poudres d'un élément de la composition de brasure et d'un composé intermétallique des éléments qui constituent la composition de brasure, par exemple le mélange des poudres de Si et de composé intermétallique ZrSi₂ pour la composition A, TiSi₂ pour la composition B, et CoSi₂ pour la composition C, constitue, dans cette seconde manière, la composition de brasure.

Ledit composé intermétallique tel que ZrSi₂, TiSi₂, ou CoSi₂, peut être synthétisé, ou bien il s'agit d'un composé du commerce se présentant sous la forme d'une poudre de composé intermétallique de granulométrie et de puretés connues.

La poudre de silicium pur peut être préparée à partir de morceaux de silicium pur broyé dans tout appareil adéquat, par exemple, dans un mortier, pour obtenir une poudre de granulométrie adéquate dont les grains ont, par exemple, un diamètre de 1 à 250 µm.

Au lieu d'être ainsi préparée, ladite poudre de silicium pur peut également être une poudre du commerce de granulométrie et de pureté connues. Parmi ces poudres du commerce, on peut citer, par exemple : la poudre de Si pur, de marque CERAC®, de pureté de 99,5% ou 99,99% et de granulométrie de l'ordre de 50 µm.

La poudre de composition de brasure est ensuite mise en suspension de manière classique dans un liant, cément, gel organique liquide, de préférence à la fois visqueux et collant, afin d'obtenir une pâte, suspension de composition de brasure éventuellement additionnée d'un renfort, par exemple en SiC, en C, ou en zircone.

Le renfort peut éventuellement améliorer le comportement mécanique de la brasure.

Il est à noter que l'étalement de la brasure est déjà excellent sans renfort.

Le liant, cément, gel se décompose généralement par exemple entre 100°C et 300°C sans laisser de traces.

Parmi les céments, on peut citer par exemple les céments disponibles auprès de la société Wall Colmonoy® sous la dénomination NICROBRAZ® tel que le cément NICROBRAZ® 650.

Parmi les gels organiques, on peut citer les gels organiques disponibles auprès de la société VITTA®.

La deuxième étape du procédé selon l'invention consiste généralement à réaliser l'assemblage par brasage proprement dit.

Au cours de cette étape, on réalise l'assemblage d'une part d'un substrat et d'autre part d'éléments, pièces discrètes que l'on peut aussi qualifier d'éléments, pièces rapportés.

Selon l'invention, au moins une partie de la surface externe du substrat et de préférence la totalité de la surface externe du substrat, à savoir la surface à assembler par brasage avec les éléments rapportés est en pyrocarbone.

Le substrat peut être constitué par du pyrocarbone ou bien le substrat peut être constitué par un cœur en un premier matériau dont au moins une partie de la surface externe du cœur en un premier matériau et de préférence la totalité de la surface externe du cœur en un premier matériau étant revêtue par un revêtement, peau, en pyrocarbone.

Le substrat recouvert d'une surface structurée obtenue par brasage des éléments rapportés est généralement utilisé comme implant ou partie d'implant destiné à être introduit dans un patient pour les prothèses de parties osseuses ou bien encore comme implant d'interposition mobile entre deux surfaces osseuses pour assurer une fonction d'articulation. De ce fait, le substrat et le cœur ont une forme qui correspond à la forme de l'implant. Cette forme dépend notamment de l'emplacement prévu pour l'implant dans le corps du patient, du fait que cet implant est soit un implant total ou partiel ou bien encore un implant d'interposition mobile.

L'invention peut être mise en œuvre avec le même succès quelle(s) que soit (soient) la forme du substrat et/ou sa taille.

Du fait que le substrat recouvert d'une surface structurée obtenue par brasage des éléments rapportés est généralement utilisé comme implant ou partie d'implant destiné à être introduit dans un patient, le cœur est généralement constitué d'un ou plusieurs premier(s) matériau(x) biocompatible(s).

A titre d'exemples de tels matériaux, on peut citer notamment la zircone, le graphite, ou le carbure de silicium.

Le revêtement de tout ou partie de la surface externe du cœur en un premier matériau par un revêtement en pyrocarbone peut être réalisé par tout procédé connu de l'homme du métier.

On pourra notamment utiliser un procédé de dépôt de pyrocarbone en lit fluidisé tel que celui décrit dans le document [3] à la description duquel on pourra se référer.

L'épaisseur de ce revêtement peut aller de 1 à 1000 µm, de préférence de 100 à 500 µm.

Ce revêtement peut être poli ou non.

Tout comme le substrat, les éléments rapportés peuvent être constitués par du pyrocarbone ou bien chacun des éléments rapportés peut être constitué par un cœur en un premier matériau au moins une partie de la surface externe du cœur en un premier matériau et de préférence la totalité de la surface externe du cœur en un premier matériau étant revêtue par un revêtement, peau, en pyrocarbone.

Le cœur de ces éléments rapportés est généralement constitué d'un ou plusieurs premier(s) matériau(x) biocompatible(s).

A titre d'exemples de tels matériaux, on peut citer notamment la zircone, le graphite, ou le carbure de silicium.

Le revêtement de la surface externe du cœur en un premier matériau des éléments rapportés par un revêtement en pyrocarbone peut être réalisé par tout procédé connu de l'homme du métier.

On pourra notamment utiliser un procédé de dépôt de pyrocarbone en lit fluidisé tel que celui décrit dans le document [3] à la description duquel on pourra se référer.

L'épaisseur de ce revêtement peut aller de 1 à 1000 µm, de préférence de 100 à 500 µm

Ce revêtement peut être poli ou non.

Les éléments rapportés peuvent se présenter sous différentes formes, par exemple sous la forme de polyèdres, de sphères, ou de billes.

De préférence, les éléments rapportés se présentent sous la forme de billes sphériques ou sphéroïdales.

Les billes peuvent avoir une granulométrie typiquement comprise entre 2,5 mm et quelques dizaines de micromètres, par exemple entre 2,5 mm et 100 µm, de préférence entre 2,5 mm et 50 µm.

Préalablement à l'assemblage, les surfaces du substrat et des éléments rapportés à assembler sont généralement dégraissées, nettoyées, dans un solvant organique par exemple du type cétone, ester, éther, alcool, ou un mélange de ceux-ci.

Un solvant préféré est l'acétone ou un mélange acétone-alcool éthylique-éther par exemple dans les proportions 1/3, 1/3, 1/3 ; on peut aussi nettoyer les surfaces successivement avec plusieurs solvants différents par exemple avec de l'acétone puis de l'éthanol. Les surfaces sont ensuite séchées.

La suspension, pâte de la composition de brasure préparée comme cela a été décrit précédemment, est étalée, enduite, appliquée de manière homogène, uniforme, par exemple avec une brosse, une spatule, un pinceau, ou à l'aide d'une seringue éventuellement fixée à un système robotisé, ou à l'aide de tout autre moyen permettant de déposer une couche de pâte de brasure uniforme, sur une partie ou sur la totalité de la surface externe en pyrocarbone du substrat tel qu'un substrat avec un cœur en zircone revêtu de pyrocarbone.

La quantité de pâte, suspension de composition de brasure est typiquement de 10 mg/cm² à 100 mg/cm², de préférence de 10 à 50 mg/cm². Cette quantité varie selon la composition de brasure.

On dépose ensuite sur la surface du substrat, ainsi préalablement revêtue d'une pâte, suspension de composition de brasure les éléments rapportés tels que des billes.

Le dépôt des pièces, éléments rapportés tels que des billes peut se faire par exemple à l'aide d'une spatule ou à l'aide d'un système robotisé permettant de déposer ces éléments rapportés tels que des billes.

La quantité de pièces, éléments rapportés déposés dépend de la taille de ces éléments rapportés.

Ainsi, dans le cas où les éléments rapportés sont des billes, la quantité de billes déposées dépend de la granulométrie des billes et peut aller de 50 mg/cm² à 300 mg/cm², de préférence de 100 mg/cm² à 150 mg/cm².

Selon l'invention, on dépose les pièces sur la composition de brasure, de façon à ce que la composition de brasure lors de sa fusion soit en contact d'une part avec la surface externe du substrat et d'autre part avec une partie à assembler de la surface externe en pyrocarbone de chacune des pièces.

C'est essentiellement voire uniquement la base en contact avec le substrat qui est en contact avec la composition de brasure. De cette manière, les pièces ne sont pas « noyées » enrobées dans la composition de brasure une fois celle-ci solidifiée et les pièces sont assemblées seulement par une de leurs surfaces telle que leur base avec le substrat.

Notons que la forme globale des pièces doit être conservée pour qu'elles puissent jouer leur rôle de structuration du substrat.

Les parties des pièces qui ne sont pas dans la zone d'assemblage peuvent être ou pas recouvertes de composition de brasure, à condition que l'épaisseur du recouvrement soit fine, reste très faible par rapport aux dimensions des pièces pour conserver leur forme globale et des espaces libres, vides pour la croissance, notamment osseuse.

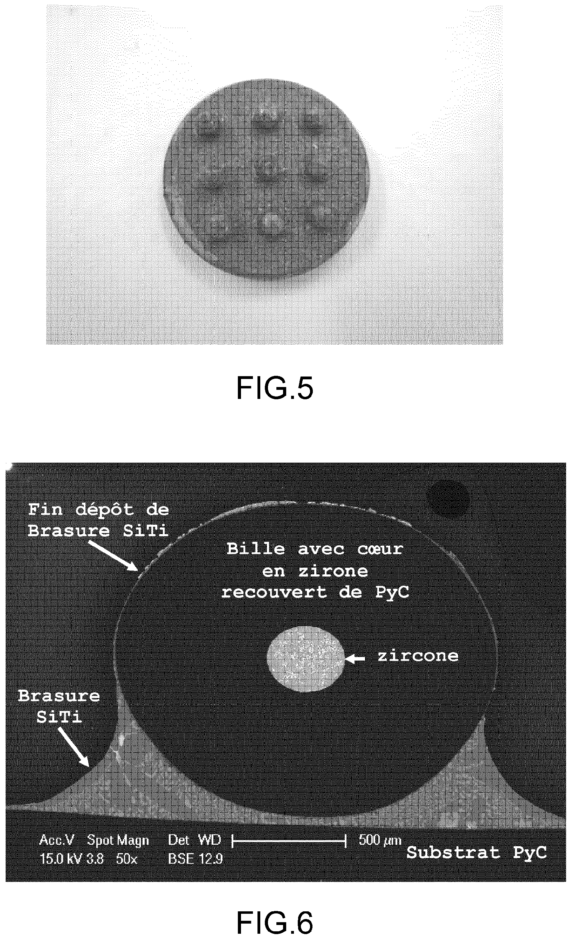

Ainsi, on considère que la couche de dépôt représentée sur la figure 6 n'est pas une bille noyée car on retrouve sa forme globale.

En outre, les éléments, pièces rapportés peuvent éventuellement avoir des points de contact entre eux, avec éventuellement formation d'un joint par ces points de contact.

Ces points de contact sont généralement discrets et d'une faible surface par rapport à la surface externe des pièces et des espaces libres, vides doivent toujours exister entre les pièces.

Ensuite, les pièces prêtes à être brasées, c'est-à-dire le substrat pourvu d'une couche de pâte de composition de brasure sur laquelle sont déposés les éléments rapportés, sont placées dans un dispositif de chauffage tel qu'un four de brasage ou soumises à un chauffage par tout autre moyen adéquat.

Le four est de préférence un four graphite et les opérations de brasage peuvent être réalisées sous vide ou sous atmosphère de gaz neutre, mais le four peut être aussi un four métallique et les opérations de brasage peuvent être de même réalisées sous vide ou sous atmosphère de gaz neutre.

Généralement, le vide est un vide secondaire, c'est-à-dire que la pression est de 10⁻³ Pa à 10⁻⁵ Pa, par exemple, de 10⁻⁴ Pa.

De préférence, le gaz neutre est de l'argon.

L'invention permet même d'utiliser de l'argon de qualité commerciale (avec généralement 5 ppm d'O₂).

Les pièces à assembler sont soumises, par exemple, dans le four à un cycle thermique sous vide, notamment sous vide secondaire, ou sous atmosphère de gaz neutre.

Ainsi, l'ensemble formé par les pièces et la composition de brasure peut-il être amené à la température de brasage en observant une montée en température de préférence « lente », avec une ou plusieurs rampes de températures depuis la température ambiante.

Cette montée en température peut se faire par exemple avec une ou plusieurs rampes de températures, chacune à raison de 1°C à 20°C/minute.

Le palier de brasage est généralement réalisé à une température, qui est la température de brasage, de préférence supérieure d'au moins 10°C, et de préférence encore d'au moins 30°C, à la température de fusion ou température de liquidus de la composition de brasure, alliage de brasage retenue.

Ainsi, la température de brasage pour les compositions de brasure, alliages de brasage A, B, C, D préférés mis en œuvre selon l'invention peut être comprise entre une température minimum de brasage de 980°C et une température de brasage maximum de 1450°C, selon la composition de brasure.

En effet, selon les compositions, la température du liquidus varie généralement de 950°C à 1420°C et la température de brasage variera donc, par exemple, comme précisé plus haut, de 980°C à 1450°C.

On a mentionné 980°C pour le point le plus bas de la température de brasage car le procédé fonctionne même avec un écart de 15°C entre la température de fusion et la température de brasage, mais un écart d'au moins 30°C est généralement préférable pour les grandes pièces, et la température de brasage la plus basse est alors de 1000°C. De même, la température de brasage la plus haute peut être de 1470°C.

Une telle température de fusion des compositions permet, selon un autre avantage du procédé de l'invention, une utilisation de l'assemblage, jusqu'à 750°C pour l'alliage D le moins réfractaire et même jusqu'à 1100°C pour l'alliage C et jusqu'à 1200°C pour les alliages A et B.

Le Tableau 2 ci-dessous indique les températures minimum et maximum de brasage pour des compositions de brasure, dont la composition, exprimée en pourcentages atomiques de silicium se situe dans les domaines de composition préférés pour les compositions de brasure, alliages de brasage, A, B, C et D.

**Tableau 2**

| **Alliage de brasage** | **Domaine de composition préféré (% at. Si)** | **Tmin de brasage (°C)** | **Tmax de brasage (°C)** |
|---|---|---|---|
| **A** | 90 à 97 | 1400°C | 1450°C |
| **B** | 83 à 88 | 1360°C | 1380°C |
| | 88 à 97 | 1380°C | 1450°C |
| **C** | 90 à 97 | 1380°C | 1450°C |
| **D** | 11 à 20 | 980°C | 1020°C |
| | 20 à 30 | 1020°C | 1200°C |
| | 30 à 50 | 1200°C | 1300°C |
| | 50 à 90 | 1300°C | 1420°C |

En mettant en œuvre une température de brasage qui se situe dans les plages définies plus haut, un bon accrochage et un très bon mouillage du pyrocarbone, avec notamment un angle de contact inferieur à 60°, sont obtenus, avec une cinétique de mouillage rapide, à savoir dès la fusion totale de la composition de brasure, comme le montrent les essais de goutte posée réalisés avec ces compositions de brasure. Ainsi, il est possible (voir exemple 1), d'obtenir un angle de contact inférieur à 60° après brasage pendant à 1420°C, avec un alliage de brasage de composition 96% de Si, et 4% de Zr en % atomiques.

Cet excellent mouillage est indispensable pour que la qualité de la liaison formée entre chaque bille et le substrat soit bonne, car il assure une bonne qualité du remplissage du joint, mais il ne permet pas toujours de garantir un bon comportement mécanique car cette dernière propriété n'est pas prévisible.

Or, de manière étonnante, les joints préparés avec des compositions de brasure selon l'invention possèdent aussi d'excellentes propriétés mécaniques.

Des essais mécaniques (voir exemples) réalisés sur des assemblages préparés selon l'invention ont montré qu'il n'y avait pas d'arrachement, pas de décohésion lors de l'essai de goutte posée, pas d'arrachement ou de décohésion des billes au refroidissement, et qu'on n'observait pas de fissures.

La température de brasage définie plus haut de 980°C à 1450°C, est maintenue pendant une durée de quelques secondes (par exemple 2, 3, 5, 10, 20, 30 secondes) à 60 minutes, de préférence de quelques secondes (par exemple 2, 3, 5, 10, 20, 30 secondes) à 20 minutes, de préférence encore de quelques secondes (par exemple 2, 3, 5, 10, 20, 30 secondes) à 5 minutes notamment pour le brasage de billes (cas des implants), c'est ce que l'on appelle le palier de brasage, ou de 10 à 20 minutes pour le brasage de surfaces à braser plus importantes que dans le cas de billes pour implants.

La durée du palier de brasage dépend de la taille des pièces à assembler et plus précisément des dimensions des surfaces à braser. On peut, en effet, aller jusqu'à 30 voire 60 minutes pour de très grandes pièces avec de grandes surfaces à braser, à savoir typiquement au moins 50x50mm².

La température spécifique du palier de brasage choisie est fonction de la composition de l'alliage de brasage.

Un palier d'homogénéisation par exemple à 1100°C à 1200°C par exemple de 1150°C pour les alliages A, B, C, ou à 750°C pour l'alliage D, est recommandé voire indispensable pour les grandes pièces (typiquement à partir de 50x50 mm²) afin de garantir l'homogénéité thermique au niveau des pièces (substrat et éléments rapportés) à assembler.

Il est à noter que la cinétique de mouillage étant déjà bonne, il n'est pas utile d'accélérer le mouillage, déjà excellent, ce premier palier est donc, dans le cas des compositions de brasure mises en œuvre selon l'invention, essentiellement voire uniquement un palier d'homogénéisation.

Ce palier peut être remplacé par une montée en température lente, par exemple à raison de seulement 0,5°C/minute, vers 1100°C pour les alliages A, B, C ou 750°C pour l'alliage D.

Les vitesses de montée en température mentionnées dans la présente dépendent de la capacité du four de brasage et de l'homogénéité thermique au sein du four.

La durée du premier palier éventuel ainsi que la durée du palier de brasage dépendent de la taille du four, des dimensions des pièces à braser et des outillages de maintien des pièces à braser.

Ce premier palier qui est donc un palier d'homogénéisation est généralement observé à une température de 1100°C à 1200°C (pour les alliages A, B, C), par exemple de 1150°C avec une durée minimale recommandée de 30 minutes, par exemple une durée de 30 à 60 minutes, avant de réaliser le palier de brasage proprement dit dans les conditions déjà mentionnées ci-dessus.

Un tel premier palier n'est pas indispensable pour les petites pièces. Un tel premier palier est généralement recommandé voire indispensable dans ces deux configurations pour les grandes pièces, à savoir généralement les pièces qui ont des surfaces à braser supérieures à 50x50 mm², afin de garantir l'homogénéité thermique au niveau des pièces à assembler.

Ou bien, une homogénéisation thermique peut également être obtenue en supprimant ce premier palier et en effectuant une montée en température lente (à raison par exemple de 0,5°C/minutes) entre généralement 1100°C et 1200°C pour les alliages A, B, C, par exemple vers 1150°C, de façon à ce que la durée d'exposition de l'assemblage dans ce domaine de température soit, par exemple, de l'ordre de 30 à 60 minutes.

Comme le premier palier, une telle montée en température lente est conseillée, voire indispensable, pour les grandes pièces.

En fin de cycle de brasage, suite au palier de brasage, on refroidit l'assemblage jusqu'à la température ambiante, en observant une ou plusieurs rampes de températures, à raison pour chacune de ces rampes par exemple de 5°C ou de 6°C par minute.

Pendant le refroidissement, la brasure se solidifie et l'assemblage des pièces en matériau à base de carbure de silicium est effectif.

L'invention va maintenant être décrite en référence à plusieurs exemples, donnés à titre illustratif et non limitatif.

### Exemples.

Dans ces exemples, on décrit des essais de goutte posée réalisés sur un substrat en zircone revêtu de pyrocarbone, et des essais d'assemblage par le procédé de brasage selon l'invention entre des disques de zircone revêtus de pyrocarbone et des billes de zircone revêtues de pyrocarbone.

### Exemple 1:

Cet exemple décrit des essais de goutte posée réalisés avec une composition de brasure, alliage de brasage selon l'invention de composition 96% de Si, 4% de Zr en % atomiques, sur un substrat en zircone en forme de disque d'un diamètre de 16 mm, revêtu par un revêtement en pyrocarbone d'une épaisseur de 300 µm.

### a) Préparation de la composition de brasure et de la pâte de brasure.

La brasure de composition visée 96% atomiques de Si et 4% atomiques de Zr, a été préparée à partir de poudre de Si et de poudre de ZrSi₂.

Ces poudres ont été pesées de façon à respecter les proportions de Zr et de Si dans la composition de la brasure et mélangées dans un « turbula ».

Un liant organique (cément 650 de NICROBRAZ® ou gel Vitta®) a été ajouté à ce mélange de poudres afin de former une pâte visqueuse.

### b) Essai de « goutte posée » à 1420°C.

La pâte de brasure ainsi préparée est utilisée pour former un petit amas de brasure d'une masse de l'ordre de 50 mg. Cet amas de brasure est déposé sur un disque d'un diamètre 16 mm en zircone avec un revêtement en pyrocarbone d'une épaisseur de 300 µm.

La plaque de zircone revêtue de pyrocarbone a été préalablement nettoyée à l'acétone puis à l'éthanol et enfin séchée.

L'ensemble de l'amas de brasure et du disque est placé dans un four de brasage, dans le cas présent un four métallique, et soumis à un cycle thermique sous vide secondaire jusqu'à un premier palier de 15 minutes à 1340°C (homogénéisation sans fusion de la brasure) puis jusqu'à un second palier à 1420°C pendant 1 mn.

L'amas de brasure fond lors de ce traitement thermique et forme une goutte que l'on appelle « goutte posée ».

Un hublot permet de suivre *in situ* l'étalement de la goutte.

L'angle de mouillage, contact, de la goutte est mesuré sur la goutte liquide *in situ* à travers le hublot.

L'angle de mouillage est inférieur à 60° dès la fusion totale de la brasure vers 1400°C, ce qui correspond à un très bon mouillage.

Après refroidissement, on observe qu'il n'y a pas de décohésion entre la goutte solidifiée et le substrat.

Ensuite, le substrat de zircone revêtu de pyrocarbone et sa goutte de brasure solidifiée ont été découpés, enrobés et polis et ont été observés par microscopie électronique à balayage.

L'absence de décohésion à l'interface est mise en évidence à l'échelle de la microscopie électronique à balayage, cette information qualitative permet de conclure que les liaisons formées sont fortes.

Notons également l'absence de fissure dans la brasure et le substrat, ce qui est favorable du point de vue du comportement mécanique.

L'interface pyrocarbone / brasure présente une réactivité très limitée à l'échelle de la microscopie électronique à balayage, réactivité qui est associée à une légère dissolution du carbone dans la brasure.

Il n'y a pas d'arrachement du substrat.

### Exemple 2 :

Cet exemple décrit la préparation de liaisons, assemblages entre des pièces de carbone revêtues de pyrocarbone d'une part, et des billes de zircone revêtues de pyrocarbone d'autre part, en mettant en œuvre le procédé de brasage selon l'invention avec une composition de brasure, alliage de brasage selon l'invention constitué de 96% de Si et de 4% de Zr, en % atomiques.

Cet exemple décrit, en outre, la caractérisation en microscopie électronique à balayage des assemblages.

### a) Préparation de la composition de brasure, de la pâte de brasure, et des pièces et billes à assembler.

La brasure de composition visée, à savoir 96% atomiques de Si et 4% atomiques de Zr, a été préparée comme décrit dans l'exemple 1.

Une pâte de composition de brasure a été formée comme dans l'exemple 1.

Les pièces en zircone revêtues de pyrocarbone sont des disques d'un diamètre de 16 mm et d'une épaisseur de 2 mm.

Les disques comportent chacun un revêtement de pyrocarbone d'une épaisseur de 300 µm de part et d'autre du disque, autrement dit sur chacune des faces du disque.

Les disques revêtus de pyrocarbone sont nettoyés à l'acétone puis à l'éthanol et enfin séchés.

Sept disques référencés n°1 à n°7 sont ainsi préparés.

Ces sept disques sont ensuite recouverts de pâte de brasure sur une de leurs faces qui est la face qui recevra les billes (avec respectivement une quantité de brasure de 13 mg, 20 mg, 21 mg, 37 mg, 80 mg, 99 mg et 170 mg).

Les billes sont ensuite déposées sur ces disques, sur la face revêtue de pâte de brasure avec une spatule.

Les billes sont des billes en zircone revêtues de pyrocarbone.

La granulométrie des billes varie entre 2 mm et 500 µm.

Le revêtement en pyrocarbone a une épaisseur de l'ordre de 300 µm à 500 µm.

Les quantités de billes déposées sur les disques n°1 à n°7 sont respectivement de 32 mg, 46 mg, 32 mg, 70 mg, 80 mg, 99 mg et 60 mg.

### b) Brasage.

Les sept ensembles disques et billes mis en contact et prêts à être brasés sont placés dans un four de brasage -qui est un four métallique dans le cas présent- et soumis à un cycle thermique sous vide secondaire jusqu'à 1420°C, un palier d'une durée de 5 minutes étant observé à cette température.

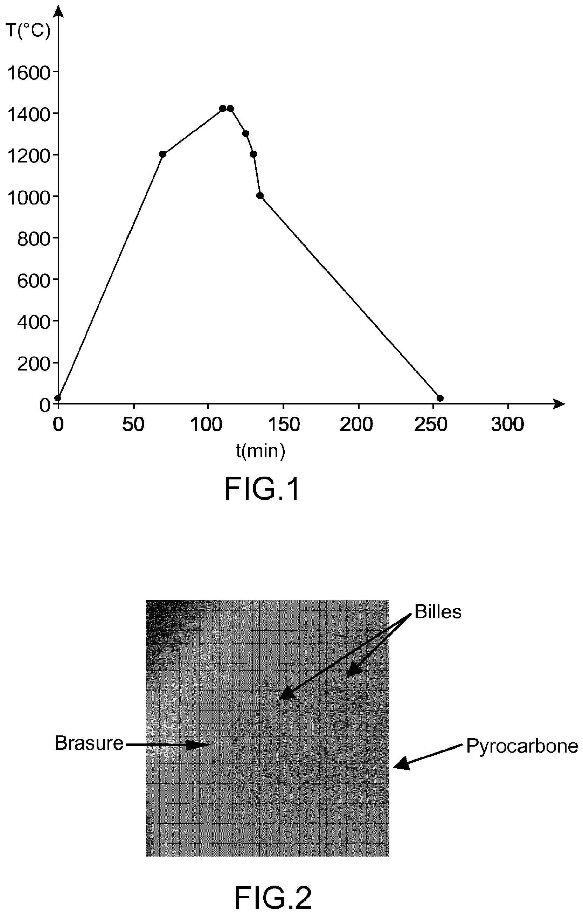

Le cycle thermique de brasage est représenté sur la Figure 1.

Le disque n°2 est placé devant le hublot du four afin de suivre *in situ* le brasage.

Au moment de la fusion, l'observation *in situ* du disque n°2 montre un bon mouillage de la base des billes par la brasure, sans recouvrement de toute la surface des billes (Figure 2).

Sur la photographie de la Figure 2, la brasure apparaît brillante entre le pyrocarbone du disque, et les billes.

### c) Observation du joint.

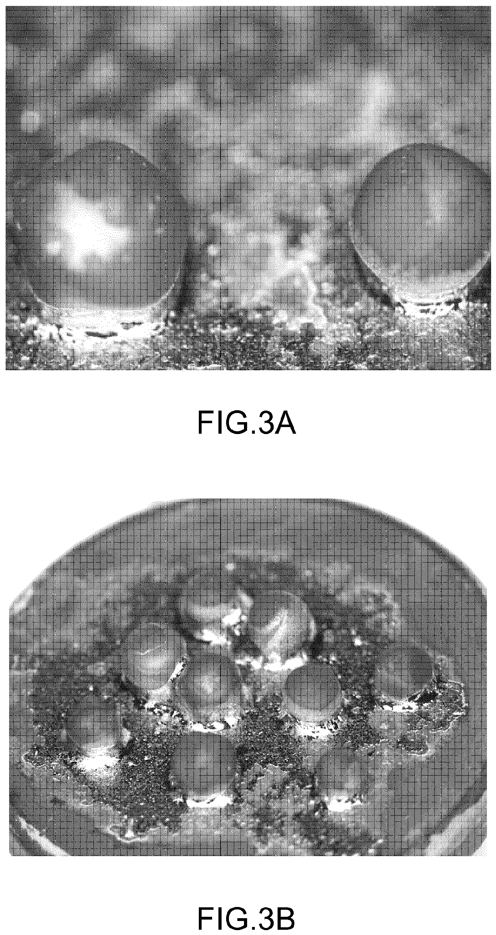

Après refroidissement, les billes sont bien assemblées sur leurs 7 disques comme le prouvent les photographies des Figures 3A et 3B qui montrent les billes assemblées sur le disque n°2.

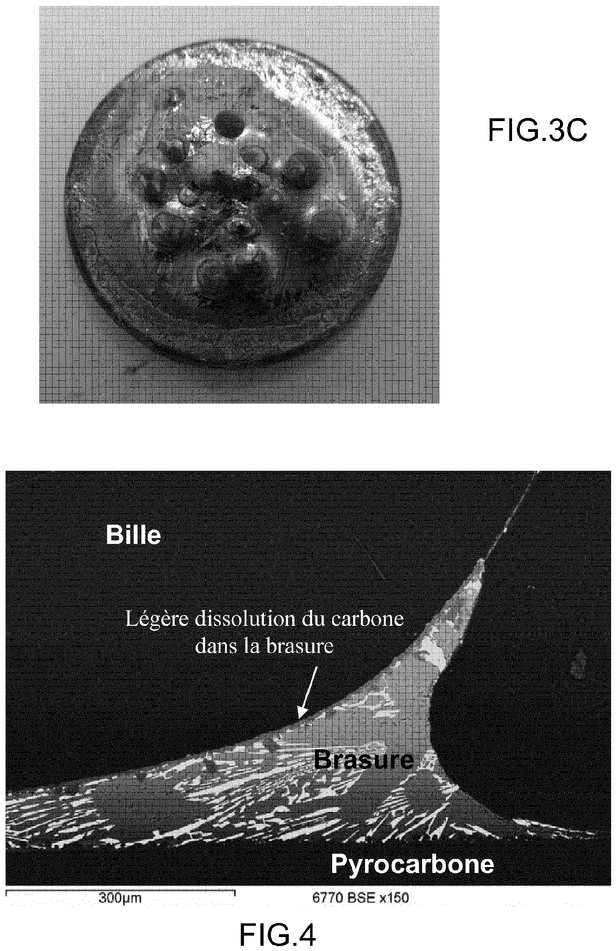

Dans le cas du disque n°7, les billes sont partiellement noyées dans la brasure comme on peut l'observer sur la Figure 3C qui montre les billes assemblées sur le disque n°7.

Les joints ont été caractérisés en microscopie électronique balayage.

Il n'y a pas de « manque » et une réactivité très limitée entre le pyrocarbone et la brasure, réactivité associée à une légère dissolution du carbone dans la brasure, est mise en évidence à l'échelle d'observation de la microcopie électronique à balayage (Figure 4).

### Exemple 3 :

Cet exemple décrit un essai de goutte posée réalisé avec une composition de brasure, alliage de brasage selon l'invention de composition 90,6% de Si, 9,4% de Ti en % atomiques, sur un substrat en zircone en forme de disque d'un diamètre de 16 mm, revêtu par un revêtement en pyrocarbone d'une épaisseur de 300 µm.

### a) Préparation de la composition de brasure et de la pâte de brasure.

La brasure de composition visée 90,6% atomiques de Si et 9,4% atomiques de Ti, a été préparée à partir de poudre de Si et de poudre de TiSi₂.

Ces poudres ont été pesées de façon à respecter les proportions de Ti et de Si dans la composition de la brasure et mélangées dans un « turbula ».

Un liant organique (cément 650 de NICROBRAZ®) a été ajouté à ce mélange de poudres afin de former une pâte visqueuse.

### b) Essai de « goutte posée » à 1420°C.

La pâte de brasure ainsi préparée est utilisée pour former un petit amas de brasure d'une masse de l'ordre de 20 mg. Cet amas de brasure est déposé sur un disque de diamètre 16 mm de zircone revêtu d'un revêtement de pyrocarbone d'une épaisseur de 300 µm.

La plaque de zircone revêtue de pyrocarbone a été préalablement nettoyée à l'acétone puis à l'éthanol et enfin séchée.

L'ensemble de l'amas de brasure et du disque est placé dans un four de brasage, dans le cas présent un four métallique, et soumis à un cycle thermique sous vide secondaire jusqu'à un unique palier de 5 minutes à 1420°C.

L'amas de brasure fond lors de ce traitement thermique et forme une goutte que l'on appelle « goutte posée ».

Un hublot permet de suivre *in situ* l'étalement de la goutte.

L'angle de mouillage, contact, de la goutte est mesuré sur la goutte liquide *in situ* à travers le hublot.

L'angle de mouillage est inférieur à 30° dès la fusion totale de la brasure. C'est un excellent mouillage.

Après refroidissement, on observe qu'il n'y a pas de décohésion entre la goutte solidifiée et le substrat.

### Exemple 4 :

Cet exemple décrit la préparation de liaisons, assemblages entre des pièces de carbone revêtues de pyrocarbone d'une part, et des billes de zircone revêtues de pyrocarbone d'autre part, en mettant en œuvre le procédé de brasage selon l'invention avec une composition de brasure, alliage de brasage selon l'invention constitué de 90,6% de Si et de 9,4% de Ti, en % atomiques.

Cet exemple décrit, en outre, la caractérisation en microscopie électronique à balayage de l'assemblage.

### a) Préparation de la composition de brasure, de la pâte de brasure, et des pièces et billes à assembler.

La brasure de composition visée, à savoir 90,6% atomiques de Si et 9,4% atomiques de Ti, a été préparée comme décrit dans l'exemple 3.

Une pâte de composition de brasure a été formée comme dans l'exemple 3.

La pièce en zircone revêtue de pyrocarbone est un disque de diamètre 16 mm et d'une épaisseur de 2 mm.

Le disque comporte un revêtement de pyrocarbone d'une épaisseur de 300 µm de part et d'autre du disque, autrement dit sur chacune des faces du disque.

Ce disque revêtu de pyrocarbone a été nettoyé à l'acétone puis à l'éthanol et enfin séché. Ce disque est ensuite recouvert de pâte de brasure sur la face qui recevra les billes avec une quantité de brasure de 42 mg.

Les billes sont ensuite déposées sur le disque, sur la face revêtue de pâte de brasure avec une spatule.

Les billes sont en zircone revêtue de pyrocarbone.

La granulométrie des billes varie entre 2 mm et 500 µm.

Le revêtement en pyrocarbone a une épaisseur de l'ordre de 300 µm à 500 µm.

La quantité de billes déposées sur le disque est 50 mg.

### b) Brasage.

L'ensemble disque et billes mis en contact et prêts à être brasés est placé dans un four de brasage -qui est un four métallique dans le cas présent- et soumis à un cycle thermique sous vide secondaire jusqu'à un unique palier à 1420°C pendant 15 minutes.

Le disque est placé devant le hublot du four afin de suivre *in situ* le brasage.

Au moment de la fusion, l'observation *in situ* du disque montre un bon mouillage de la base des billes par la brasure avec un léger recouvrement de la surface des billes.

### c) Observation du joint.

Après refroidissement, les billes sont bien assemblées sur le disque comme le montre la photographie de la Figure 5.

Les joints ont été caractérisés en microscopie électronique à balayage.

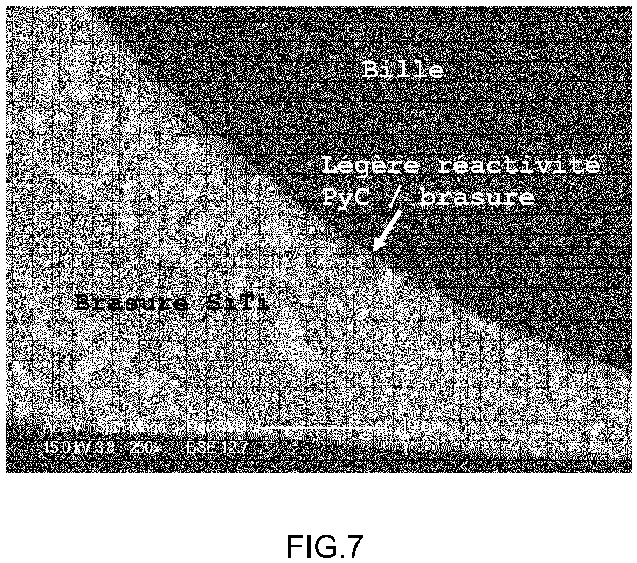

Il n'y a pas de « manque ». Une réactivité très limitée est observée entre le pyrocarbone et la brasure, réactivité associée à une légère dissolution du carbone dans la brasure. Cette réactivité est mise en évidence à l'échelle d'observation de la microscopie électronique à balayage (Figures 6 et 7). Un léger recouvrement des billes est observé.

Plus précisément, sur la Figure 6, on observe une bille avec un cœur en zircone recouvert de Pyrocarbone (PyC), le substrat en pyrocarbone, la brasure en SiTi entre la bille et le substrat, et un fin dépôt de brasure SiTi sur la surface de la bille.

Sur la Figure 7, est mise en évidence une légère réactivité entre le pyrocarbone de la bille et la brasure et entre le pyrocarbone du substrat et la brasure.

### Exemple 5 :

Cet exemple décrit la préparation d'une liaison, assemblage entre une pièce de carbone revêtue de pyrocarbone d'une part, et une bille de zircone revêtue de pyrocarbone d'autre part, en mettant en œuvre le procédé de brasage selon l'invention avec une composition de brasure, alliage de brasage selon l'invention constitué de 90,6% de Si et de 9,4% de Ti, en % atomiques.

### a) Préparation de la composition de brasure, de la pâte de brasure, et des pièces et bille à assembler.

La brasure de composition visée, à savoir 90,6% atomiques de Si et 9,4% atomiques de Ti, a été préparée comme décrit dans l'exemple 3.

Une pâte de composition de brasure a été formée comme dans l'exemple 3.

La pièce en zircone revêtue de pyrocarbone est un disque de diamètre 16 mm et d'une épaisseur de 2 mm.

Le disque comporte un revêtement de pyrocarbone d'une épaisseur de 300 µm de part et d'autre du disque, autrement dit sur chacune des faces du disque.

Ce disque revêtu de pyrocarbone a été nettoyé à l'acétone puis à l'éthanol et enfin séché. Ce disque est ensuite recouvert sur une zone de 5x5 mm² de pâte de brasure sur la face qui recevra la bille avec respectivement une quantité de brasure de 2 mg pour cette unique bille.

La bille est ensuite déposée sur le disque, sur la zone revêtue de pâte de brasure avec une spatule.

La bille est en zircone revêtue de pyrocarbone.

La granulométrie de bille est comprise entre 2 mm et 500 µm.

Le revêtement en pyrocarbone a une épaisseur de l'ordre de 300 µm à 500 µm.

### b) Brasage.

L'ensemble disque et bille mis en contact et prêts à être brasés est placé dans un four de brasage -qui est un four métallique dans le cas présent- et soumis à un cycle thermique sous argon jusqu'à un unique palier à 1320°C pendant 5 minutes.

Le disque est placé devant le hublot du four afin de suivre *in situ* le brasage.

Au moment de la fusion, l'observation *in situ* du disque montre un bon mouillage de la base de la bille par la brasure avec un recouvrement partiel de la surface de la bille.

### c) Observation de la bille.

Après refroidissement, la bille est bien assemblée sur le disque. Le dessus de la bille a été caractérisé en microscopie électronique balayage. On observe un recouvrement partiel de la bille.

### Exemple 6 :

Cet exemple décrit des essais de goutte posée réalisés avec une composition de brasure, alliage de brasage selon l'invention de composition 77,5% de Si, 22,5% de Co en % atomiques, sur un substrat en zircone en forme de disque d'un diamètre de 16 mm, revêtu d'un revêtement en pyrocarbone d'une épaisseur de 300 µm.

### a) Préparation de la composition de brasure et de la pâte de brasure.

La brasure de composition visée 77,5% atomiques de Si et 22,5% atomiques de Co, a été préparée à partir de poudre de Si et de poudre de CoSi₂.

Ces poudres ont été pesées de façon à respecter les proportions de Co et de Si dans la composition de la brasure et mélangées dans un « turbula ».

Un liant organique (cément 650 de NICROBRAZ®) a été ajouté à ce mélange de poudres afin de former une pâte visqueuse.

### b) Essai de « goutte posée » à 1320°C.

La pâte de brasure ainsi préparée est utilisée pour former un petit amas de brasure d'une masse de l'ordre de 50 mg. Cet amas de brasure est déposé sur un disque de diamètre 16 mm de zircone revêtu de pyrocarbone.

La plaque de zircone revêtue de pyrocarbone a été préalablement nettoyée à l'acétone puis à l'éthanol et enfin séchée.

L'ensemble de l'amas de brasure et du disque est placé dans un four de brasage, dans le cas présent un four métallique, et soumis à un cycle thermique sous vide secondaire jusqu'à un unique palier de 5 minutes à 1320°C.

L'amas de brasure fond lors de ce traitement thermique et forme une goutte que l'on appelle « goutte posée ».

Un hublot permet de suivre *in situ* l'étalement de la goutte.

L'angle de mouillage, contact, de la goutte est mesuré sur la goutte liquide *in situ* à travers le hublot.

L'angle de mouillage est inférieur à 60° dès la fusion totale de la brasure, c'est un bon mouillage.

Après refroidissement, on observe qu'il n'y a pas de décohésion entre la goutte solidifiée et le substrat.

### Exemple 7 :

Cet exemple décrit la préparation de liaisons, assemblages entre des pièces de carbone revêtues de pyrocarbone d'une part, et des billes de zircone revêtues de pyrocarbone d'autre part, en mettant en œuvre le procédé de brasage selon l'invention avec une composition de brasure, alliage de brasage selon l'invention constitué de 77,5% de Si et de 22,5% de Co, en % atomiques.

Cet exemple décrit, en outre, la caractérisation en microscopie électronique à balayage des assemblages.

### a) Préparation de la composition de brasure, de la pâte de brasure, et des pièces et billes à assembler.

La brasure de composition visée, à savoir 77,5% atomiques de Si et 22,5% atomiques de Co, a été préparée comme décrit dans l'exemple 6.

Une pâte de composition de brasure a été formée comme dans l'exemple 6.

Les pièces en zircone revêtues de pyrocarbone sont des disques d'un diamètre de 16 mm et d'une épaisseur de 2 mm.

Les disques comportent chacun un revêtement de pyrocarbone d'une épaisseur de 300 µm de part et d'autre du disque, autrement dit sur chacune des faces du disque.

Le disque revêtu de pyrocarbone a été nettoyé à l'acétone puis à l'éthanol et enfin séché.

Ce disque a ensuite été recouvert de pâte de brasure sur la face qui recevra les billes avec une quantité de brasure de 29 mg.

Les billes sont ensuite déposées sur ces disques, sur la face revêtue de pâte de brasure avec une spatule.

Les billes sont en zircone revêtue de pyrocarbone.

La granulométrie des billes varie entre 2 mm et 500 µm.

Le revêtement en pyrocarbone a une épaisseur de l'ordre de 300 µm à 500 µm.

La quantité de billes déposées sur le disque est 45 mg.

### b) Brasage.

L'ensemble disque et billes mis en contact et prêt à être brasé est placé dans un four de brasage -qui est un four métallique dans le cas présent- et soumis à un cycle thermique sous argon jusqu'à 1270°C pendant 30 secondes.

Le disque est placé devant le hublot du four afin de suivre *in situ* le brasage.

Au moment de la fusion, l'observation *in situ* du disque montre un bon mouillage de la base des billes par la brasure, sans recouvrement de toute la surface des billes.

### c) Observation du joint.

Après refroidissement, les billes sont bien assemblées sur le disque.

Les joints ont été caractérisés en microscopie électronique balayage.

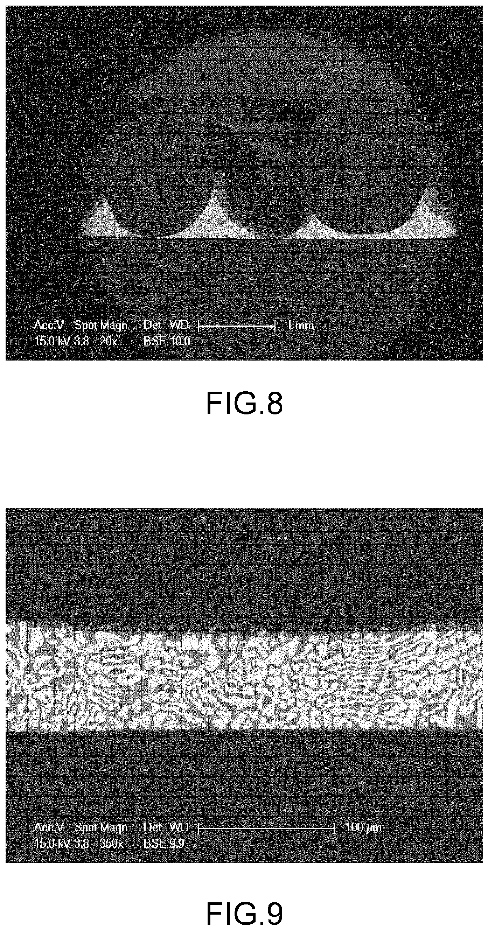

Il n'y a pas de « manque » et une réactivité très limitée entre le pyrocarbone et la brasure, réactivité associée à une légère dissolution du carbone dans la brasure, est mise en évidence à l'échelle d'observation de la microcopie électronique à balayage (Figures 8 à 10).

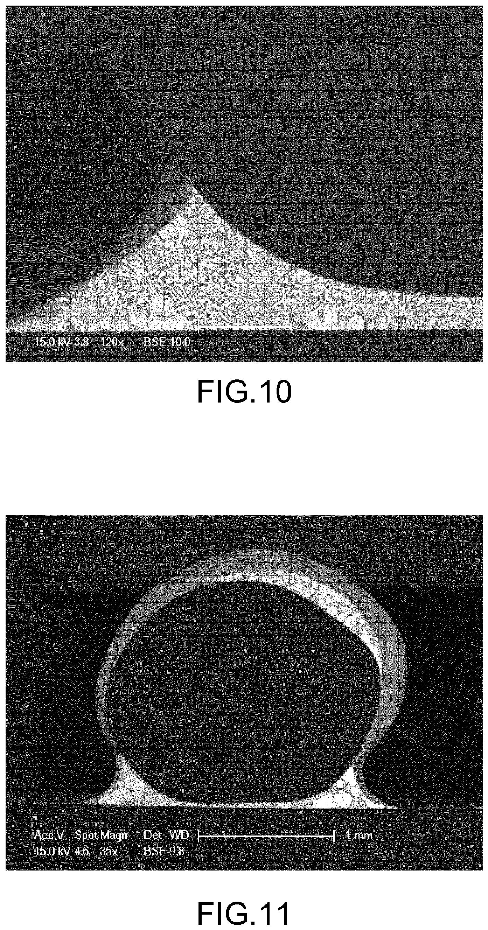

Plus précisément, la Figure 8 montre l'assemblage entre deux billes et le substrat, et les Figures 9 et 10 montrent le joint entre une bille et le substrat.

### Exemple 8 :

Cet exemple décrit la préparation de liaisons, assemblages entre des pièces de carbone revêtues de pyrocarbone d'une part, et des billes de zircone revêtues de pyrocarbone d'autre part, en mettant en œuvre le procédé de brasage selon l'invention avec une composition de brasure, alliage de brasage selon l'invention constitué de 77,5% de Si et de 22,5% de Co, en % atomiques.

Cet exemple décrit, en outre, la caractérisation en microscopie électronique à balayage des assemblages.

### a) Préparation de la composition de brasure, de la pâte de brasure, et des pièces et billes à assembler.

La brasure de composition visée, à savoir 77,5% atomiques de Si et 22,5% atomiques de Co, a été préparée comme décrit dans l'exemple 6.

Une pâte de composition de brasure a été formée comme dans l'exemple 6.

Les pièces en zircone revêtue de pyrocarbone sont des disques d'un diamètre de 16 mm et d'une épaisseur de 2 mm.

Les disques comportent chacun un revêtement de pyrocarbone d'une épaisseur de 300 µm de part et d'autre du disque, autrement dit sur chacune des faces du disque.

Le disque revêtu de pyrocarbone a été nettoyé à l'acétone puis à l'éthanol et enfin séché.

Ce disque a ensuite été recouvert de pâte de brasure sur la face qui recevra les billes avec une quantité de brasure de 29 mg.

Les billes sont ensuite déposées sur ces disques, sur la face revêtue de pâte de brasure avec une spatule.

Les billes sont en zircone revêtue de pyrocarbone.

La granulométrie des billes varie entre 2 mm et 500 µm.

Le revêtement en pyrocarbone a une épaisseur de l'ordre de 300 µm à 500 µm.

La quantité de billes déposées sur le disque est 45 mg.

### b) Brasage.

L'ensemble disque et billes mis en contact et prêt à être brasé est placé dans un four de brasage -qui est un four métallique dans le cas présent- et soumis à un cycle thermique sous argon jusqu'à 1320°C pendant 5 minutes.

Le disque est placé devant le hublot du four afin de suivre *in situ* le brasage.

Au moment de la fusion, l'observation *in situ* du disque montre un bon mouillage des billes par la brasure avec un recouvrement de toute la surface des billes.

### c) Observation du joint.

Après refroidissement, les billes sont bien assemblées sur le disque.

Les billes et assemblages avec le substrat ont été caractérisés en microscopie électronique balayage.

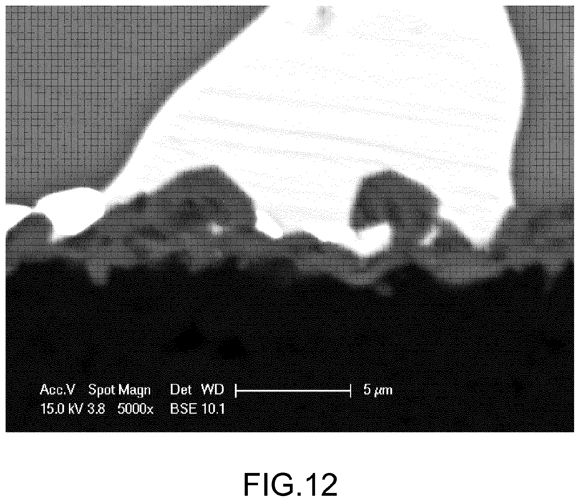

Il n'y a pas de « manque » et une réactivité très limitée entre le pyrocarbone et la brasure, réactivité associée à une légère dissolution du carbone dans la brasure, est mise en évidence à l'échelle d'observation de la microcopie électronique à balayage (Figures 11 et 12).

Plus précisément, la Figure 11 montre l'assemblage entre une bille et le substrat, et la Figure 12 montre l'interface entre le pyrocarbone et la brasure et met en évidence une légère réactivité (zone de réaction en gris sur la Figure 12).

### Exemple 9 :

Des essais de cytotoxicité, biocompatibilité, ont été réalisés sur des échantillons de pyrocarbone revêtu de brasures Zr-Si, Co-Si, et Ti-Si.

Ces essais ont été réalisés selon la norme ISO 10993 avec des cultures de cellule de fibroblaste de souris, L-929.

Aucune destruction cellulaire, ni aucune toxicité des échantillons sur les cellules n'a été mise en évidence lors de ces essais.

### RÉFÉRENCES

[1] FR-A1-2 893 247
[2] EP-A1-1 112 753
[3] WO-A1-01/32950
[4] WO-A1-01/68557
[5] WO-A1-01/68560

## Revendications

1. Procédé d'assemblage par brasage d'un substrat comprenant au moins une surface externe en pyrocarbone avec des pièces comprenant chacune au moins une surface externe en pyrocarbone, dans lequel on réalise les étapes successives suivantes :
- on dépose une composition de brasure constituée par un alliage d'un ou plusieurs métal (métaux) ou métalloïde(s) sur au moins une partie à assembler de la surface externe en pyrocarbone du substrat ;
- on dépose les pièces sur la composition de brasure, de façon à ce que la composition de brasure, lors de sa fusion, soit en contact d'une part avec la partie à assembler de la surface externe du substrat et d'autre part avec une partie à assembler de la surface externe en pyrocarbone de chacune des pièces, de façon à ce que la forme des pièces ne soit pas modifiée et reste apparente, de façon à laisser des espaces libres, vides entre les pièces, et de façon à ce que les pièces ne soient pas noyées dans la composition de brasure ;
- on chauffe l'ensemble formé par le substrat, la composition de brasure et les pièces déposées, sous vide ou sous une atmosphère de gaz neutre, à une température de brasage suffisante pour faire fondre totalement ou au moins partiellement la composition de brasure, et inférieure à 1700°C ;
- on refroidit le substrat, la composition de brasure et les pièces jusqu'à solidification de la composition de brasure, procédé dans lequel la dite composition de brasure est choisie parmi les alliages à base de silicium contenant du Si à une concentration supérieure à 40% atomiques et qui présentent une réactivité limitée et maîtrisée avec le pyrocarbone.

2. Procédé selon la revendication 1, dans lequel deux ou plus parmi les pièces déposées sont en contact entre elles ou bien toutes les pièces déposées ne sont pas en contact entre elles.

3. Procédé selon la revendication 1, dans lequel le brasage est effectué à une température de brasage supérieure d'au moins 15°C, de préférence d'au moins 30°C à la température de fusion de la brasure.

4. Procédé selon l'une quelconque des revendications précédentes, dans lequel la température de brasage est de 980°C à 1450°C.

5. Procédé selon l'une quelconque des revendications 1 à 4, dans lequel ladite composition de brasure est choisie parmi les alliages binaires constitués par du silicium à une concentration supérieure à 40% atomiques et par un élément choisi parmi Cr, Re, V, Ru, Ir, Rh, Pd, Co, Pt, Ce, Zr, Ti, Ag, Au, Cu, Nd, Pr, Y, et Hf.

6. Procédé selon la revendication 5, dans lequel ladite composition de brasure est choisie parmi les alliages binaires (A) constitués de silicium et de zirconium, les alliages binaires (B) constitués de silicium et de titane, les alliages binaires (C) constitués de silicium et de cobalt, et les alliages binaires (D) constitués de silicium et d'argent.

7. Procédé selon la revendication 6, dans lequel ladite composition de brasure est un alliage binaire (A) constitué en pourcentages atomiques de 75% à 97% de silicium, et de 25% à 3% de Zr; de préférence de 89% à 97% de silicium, et de 11% à 3% de zirconium ;ou ladite composition de brasure est un alliage binaire (B) constitué en pourcentages atomiques de 60% à 97% de silicium, et de 40% à 3% de Ti ; de préférence de 80% à 97% de silicium, et de 20% à 3% de titane ; ou ladite composition de brasure est un alliage binaire (C) constitué en pourcentages atomiques de 60% à 97% de silicium, et de 40% à 3% de Co ; de préférence de 75% à 97% de silicium, et de 25% à 3% de cobalt ; ou ladite composition de brasure est un alliage binaire (D) constitué en pourcentages atomiques de plus de 40% jusqu'à 97% de silicium, et de moins de 60 % jusqu'à 3% d'Ag; de préférence de plus de 40% jusqu'à 90% de silicium, et de moins de 60 % à 10% d'argent.

8. Procédé selon l'une quelconque des revendications précédentes, dans lequel le substrat est constitué par du pyrocarbone, ou bien le substrat est constitué par un cœur en un premier matériau choisi de préférence parmi les matériaux biocompatibles tels que la zircone, le graphite, ou le carbure de silicium ; au moins une partie de la surface externe du cœur en un premier matériau et de préférence la totalité de la surface externe du cœur en un premier matériau étant revêtue par un revêtement ou peau en pyrocarbone.

9. Procédé selon l'une quelconque des revendications précédentes, dans lequel les pièces sont constituées par du pyrocarbone ou bien chacune des pièces est constituée par un cœur en un premier matériau choisi de préférence parmi les matériaux biocompatibles tels que la zircone, le graphite, ou le carbure de silicium ; au moins une partie de la surface externe du cœur en un premier matériau et de préférence la totalité de la surface externe du cœur en un premier matériau étant revêtue par un revêtement ou peau en pyrocarbone.

10. Procédé selon l'une quelconque des revendications précédentes, dans lequel les pièces se présentent sous la forme de billes sphériques ou sphéroïdales, de préférence avec une granulométrie comprise entre 2,5 mm et quelques dizaines de micromètres, par exemple entre 2,5 mm et 100 µm, de préférence entre 2,5 mm et 50 µm.

11. Procédé selon l'une quelconque des revendications précédentes, dans lequel on forme une poudre de composition de brasure, on met en suspension ladite poudre dans un liant organique de façon à obtenir une suspension ou pâte de composition de brasure, et on dépose ladite suspension ou pâte sur au moins une partie à assembler de la surface externe en pyrocarbone du substrat.

12. Procédé selon l'une quelconque des revendications précédentes, dans lequel ledit substrat est un implant, ou partie d'implant, pour prothèses de parties osseuses ; ou un implant d'interposition entre des surfaces osseuses.

13. Assemblage obtenu par le procédé selon l'une quelconque des revendications 1 à 12.

## Patentansprüche

1. Verfahren zum Verlöten eines Substrats, das zumindest eine Außenfläche aus Pyrokohlenstoff aufweist, mit Teilen, die jeweils zumindest eine Außenfläche aus Pyrokohlenstoff aufweisen, wobei die nachstehenden aufeinanderfolgenden Schritte ausgeführt werden:
- Aufbringen einer Lotzusammensetzung, die aus einer Legierung aus einem oder mehreren Metall(en) oder Metalloid(en) besteht, auf zumindest einen anzufügenden Abschnitt der Pyrokohlenstoff-Außenfläche des Substrats;
- Aufbringen der Teile auf die Lotzusammensetzung derart, dass die Lotzusammensetzung während ihres Schmelzens einerseits mit dem anzufügenden Abschnitt der Außenfläche des Substrats und andererseits mit einem anzufügenden Abschnitt der Pyrokohlenstoff-Außenfläche jedes der Teile in Kontakt ist, dass die Form der Teile nicht verändert wird und erkennbar bleibt, dass freie Leerräume zwischen den Teilen verbleiben und dass die Teile nicht in der Lotzusammensetzung versenkt werden;
- Erhitzen der aus dem Substrat, der Lotzusammensetzung und den aufgebrachten Teilen gebildeten Anordnung unter Vakuum oder unter eine Inertgasatmosphäre auf eine Löttemperatur, die ausreicht, um die Lotzusammensetzung vollständig oder zumindest teilweise zu schmelzen, und die unter 1700°C liegt;
- Abkühlen des Substrats, der Lotzusammensetzung und der Teile, bis die Lotzusammensetzung erstarrt ist, wobei bei dem Verfahren die Lotzusammensetzung aus Legierungen auf Siliciumbasis ausgewählt ist, die Si in einer Konzentration von über 40 Atomprozent enthalten und die eine begrenzte und kontrollierte Reaktivität mit Pyrokohlenstoff aufweisen.

2. Verfahren nach Anspruch 1,
wobei zwei oder mehrere unter der aufgebrachten Teile miteinander in Kontakt stehen oder nicht alle aufgebrachten Teile miteinander in Kontakt stehen.

3. Verfahren nach Anspruch 1,
wobei das Löten bei einer Löttemperatur erfolgt, die zumindest um 15°C, vorzugsweise zumindest um 30°C, höher als die Schmelztemperatur des Lotes liegt.

4. Verfahren nach einem der vorangehenden Ansprüche,
wobei die Löttemperatur 980°C bis 1450°C beträgt.

5. Verfahren nach einem der Ansprüche 1 bis 4,
wobei die Lotzusammensetzung aus binären Legierungen ausgewählt ist, die aus Silicium in einer Konzentration von über 40 Atom-% und einem Element, ausgewählt aus Cr, Re, V, Ru, Ir, Rh, Pd, Co, Pt, Ce, Zr, Ti, Ag, Au, Cu, Nd, Pr, Y und Hf, bestehen.

6. Verfahren nach Anspruch 5,
wobei die Lotzusammensetzung ausgewählt ist aus binären Legierungen (A), die aus Silicium und Zirkonium bestehen, binären Legierungen (B), die aus Silicium und Titan bestehen, binären Legierungen (C), die aus Silicium und Kobalt bestehen, und binären Legierungen (D), die aus Silicium und Silber bestehen.

7. Verfahren nach Anspruch 6,
wobei die Lotzusammensetzung eine binäre Legierung (A) ist, die zu 75 bis 97 Atom-% aus Silicium und zu 25 bis 3 Atom-% aus Zr besteht, vorzugsweise zu 89 bis 97 Atom-% aus Silicium und zu 11 bis 3 Atom-% aus Zirkonium; oder wobei die Lotzusammensetzung eine binäre Legierung (B) ist, die zu 60 bis 97 Atom-% aus Silicium und zu 40 bis 3 Atom-% aus Ti besteht; vorzugsweise zu 80 bis 97 Atom-% aus Silicium und zu 20 bis 3 Atom-% aus Titan; oder
wobei die Lotzusammensetzung eine binäre Legierung (C) ist, die zu 60 bis 97 Atom-% aus Silicium und zu 40 bis 3 Atom-% aus Co besteht; vorzugsweise zu 75 bis 97 Atom-% aus Silicium und zu 25 bis 3 Atom-% aus Kobalt; oder
wobei die Lotzusammensetzung eine binäre Legierung (D) ist, die zu über 40 bis 97 Atom-% aus Silicium und zu unter 60 bis 3 Atom-% aus Ag besteht; vorzugsweise zu über 40 bis 90 Atom-% aus Silicium und zu unter 60 bis 10 Atom-% aus Silber.

8. Verfahren nach einem der vorangehenden Ansprüche,
wobei das Substrat aus Pyrokohlenstoff besteht oder das Substrat aus einem Kern aus einem ersten Material besteht, das vorzugsweise aus biokompatiblen Materialien wie Zirkoniumdioxid, Graphit oder Siliciumcarbid ausgewählt ist; wobei zumindest ein Abschnitt der Außenfläche des Kerns aus einem ersten Material und vorzugsweise die gesamte Außenfläche des Kerns aus einem ersten Material mit einer Beschichtung oder Haut aus Pyrokohlenstoff beschichtet ist.

9. Verfahren nach einem der vorangehenden Ansprüche,
wobei die Teile aus Pyrokohlenstoff bestehen oder jedes der Teile aus einem Kern aus einem ersten Material besteht, das vorzugsweise aus biokompatiblen Materialien wie Zirkoniumdioxid, Graphit oder Siliciumcarbid ausgewählt ist; wobei zumindest ein Abschnitt der Außenfläche des Kerns aus einem ersten Material und vorzugsweise die gesamte Außenfläche des Kerns aus einem ersten Material mit einer Beschichtung oder Haut aus Pyrokohlenstoff beschichtet ist.

10. Verfahren nach einem der vorangehenden Ansprüche,
wobei die Teile in Form von sphärischen oder sphäroidalen Kugeln vorliegen, vorzugsweise mit einer Korngröße zwischen 2,5 mm und einigen zehn Mikrometern, beispielsweise zwischen 2,5 mm und 100 µm, vorzugsweise zwischen 2,5 mm und 50 µm.

11. Verfahren nach einem der vorangehenden Ansprüche,
wobei ein Pulver aus Lotzusammensetzung gebildet wird, das Pulver in einem organischen Bindemittel in Suspension gebracht wird, um eine Suspension oder Paste der Lotzusammensetzung zu erhalten, und die Suspension oder Paste auf zumindest einen anzufügenden Abschnitt der Pyrokohlenstoff-Außenfläche des Substrats aufgebracht wird.

12. Verfahren nach einem der vorangehenden Ansprüche,
wobei das Substrat ein Implantat oder ein Teil eines Implantats für Prothesen von Knochenteilen; oder ein Implantat für die Einfügung zwischen Knochenoberflächen ist.

13. Zusammenfügung, die durch das Verfahren nach einem der Ansprüche 1 bis 12 erhalten wird.

## Claims

1. A method for assembling by brazing a substrate comprising at least one external pyrocarbon surface with parts each comprising at least one external pyrocarbon surface, wherein the following successive steps are carried out:
- a brazing material composition consisting of an alloy of one or several metal(s) or metalloid(s) is deposited on at least one portion to be assembled of the external pyrocarbon surface of the substrate;
- the parts are deposited on the brazing material composition, so that the brazing material composition upon its melting, is in contact with the portion to be assembled of the external surface of the substrate on the one hand, and with a portion to be assembled of the external pyrocarbon surface of each of the parts of the other hand, so that the shape of the parts is not modified and remains apparent, so as to leave free, empty spaces between the parts, and so that the parts are not embedded in the brazing material composition;
- the assembly formed by the substrate, the brazing material composition and the deposited parts is heated *in vacuo* or in a neutral gas atmosphere at a brazing temperature sufficient for totally or at least partially melting the brazing material composition, and less than 1,700°C;
- the substrate, the brazing material composition and the parts are cooled until solidification of the brazing material composition, method wherein said brazing material composition is selected from alloys based on silicon containing Si at a concentration of more than 40 atomic %, and which have limited and controlled reactivity with pyrocarbon.

2. The method according to claim 1, wherein two or more from among the deposited parts are in contact with each other or else all the deposited parts are not in contact with each other.

3. The method according to claim 1, wherein the brazing is carried out at a brazing temperature greater by at least 15°C, preferably by at least 30°C than the melting temperature of the brazing material.

4. The method according to any one of the preceding claims, wherein the brazing temperature is from 980°C to 1,450°C.

5. The method according to any one of claims 1 to 4, wherein said brazing material composition is selected from binary alloys consisting of silicon at a concentration of more than 40 atomic % and of an element selected from Cr, Re, V, Ru, Ir, Rh, Pd, Co, Pt, Ce, Zr, Ti, Ag, Au, Cu, Nd, Pr, Y and Hf.

6. The method according to claim 5, wherein said brazing material composition is selected from binary alloys (A) consisting of silicon and zirconium, binary alloys (B) consisting of silicon and titanium, binary alloys (C) consisting of silicon and cobalt, and binary alloys (D) consisting of silicon and silver.

7. The method according to claim 6, wherein said brazing composition is a binary alloy (A) consisting of 75% to 97% silicon and of 25% to 3% Zr; preferably from 89% to 97% silicon, and from 11% to 3% zirconium in atomic percentages; or said brazing composition is a binary alloy (B) consisting of 60% to 97% silicon, and from 40% to 3% Ti; preferably from 80% to 97% silicon, and from 20% to 3% titanium in atomic percentages; or said brazing material composition is a binary alloy (C) consisting of 60% to 97% silicon, and of 40% to 3% Co; preferably from 75% to 97% silicon, and from 25% to 3% cobalt, in atomic percentages; or said brazing material composition is a binary alloy (D) consisting of more than 40% to 97% silicon, and of less than 60% to 3% Ag; preferably from more than 40% to 90% silicon and from less than 60% to 10% silver, in atomic percentages.

8. The method according to any one of the preceding claims, wherein the substrate consists of pyrocarbon, or else the substrate consists of a core made of a first material preferably selected from biocompatible materials such as zirconia, graphite or silicon carbide; at least one portion of the external surface of the core made of a first material and preferably the totality of the external surface of the core made of a first material being coated with a pyrocarbon coating or skin.

9. The method according to any one of the preceding claims, wherein the parts consist of pyrocarbon or else each of the parts consist of a core made of a first material preferably selected from biocompatible materials such as zirconia, graphite or silicon carbide; at least one portion of the external surface of the core made of a first material and preferably the totality of the external surface of the core made of a first material being coated with a pyrocarbon coating or skin.

10. The method according to any one of the preceding claims, wherein the parts appear as spherical or spheroidal beads, preferably with a grain size comprised between 2.5 mm and a few tens of micrometers, for example between 2.5 mm and 100 µm, preferably between 2.5 mm and 50 µm.

11. The method according to any one of the preceding claims, wherein a brazing material composition powder is formed, said powder is suspended in an organic binder so as to obtain a brazing material composition suspension or slurry, paste and said suspension or slurry, paste is deposited on at least one portion to be assembled of the external pyrocarbon surface of the substrate.

12. The method according to any one of the preceding claims, wherein said substrate is an implant or part of an implant, for prostheses of bone portions; or an interposition implant between bone surfaces.

13. The assembly obtained by the method according to any one of claims 1 to 12.
